(19) Europäisches Patentamt
European Patent Office
Office européen des brevets

(11) **EP 3 244 211 A1**

(12) **EUROPEAN PATENT APPLICATION**
published in accordance with Art. 153(4) EPC

(43) Date of publication:
**15.11.2017 Bulletin 2017/46**

(51) Int Cl.:
*G01N 33/569* (2006.01)  *G01N 33/58* (2006.01)
*G01N 21/78* (2006.01)  *C12Q 1/37* (2006.01)

(21) Application number: **16735193.1**

(22) Date of filing: **08.01.2016**

(86) International application number:
**PCT/KR2016/000163**

(87) International publication number:
**WO 2016/111572 (14.07.2016 Gazette 2016/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **08.01.2015 KR 20150002793
19.01.2015 KR 20150008955
19.01.2015 KR 20150008956
07.01.2016 KR 20160002305**

(71) Applicants:
• **Huvet Bio, Inc.
Seoul 06247 (KR)**

• **Korea Research Institute of Bioscience and
Biotechnology
Daejeon 34141 (KR)**

(72) Inventors:
• **HAAM, Seungjoo
Seoul 03722 (KR)**
• **KIM, Hyun-Ouk
Seoul 03722 (KR)**
• **KIM, Jihye
Seoul 03722 (KR)**
• **SONG, Daesub
Daejeon 34069 (KR)**

(74) Representative: **Vossius & Partner
Patentanwälte Rechtsanwälte mbB
Siebertstrasse 3
81675 München (DE)**

(54) **KIT FOR DETECTING VIRUS**

(57)    The present invention relates to a kit for detecting a virus, a composition for detecting a virus and a method for detecting a virus. According to the present invention, a kit which is capable of detecting viruses with high efficiency at low cost within a short period of time, and exhibits enhanced sensitivity and accuracy may be provided.

【FIG. 1】

**Description**

[Technical Field]

The present invention relates to a kit for detecting a virus, a composition for detecting a virus, and a method for detecting a virus.

[Background Art]

Viruses are contagious pathogens smaller than bacteria. A virus consists of RNA or DNA as a genetic material and a protein surrounding the genetic material. Viruses may be classified into plant viruses, animal viruses and bacterial viruses (phages) according to the type of host. However, in most cases, viruses are divided into DNA virus subphyla and RNA virus subphyla according to the type of nucleic acid, and also subdivided into class, order and family.

Among such viruses, avian influenza is an acute viral infectious disease caused by an infection by avian influenza viruses in chickens, ducks or wild birds, and rarely developing infectious diseases in humans. Avian influenza viruses are classified into three types of high pathogenicity, low pathogenicity and non-pathogenicity according to pathogenicity, and over 640 cases of human infections by the highly pathogenic Avian influenza A (H5N1) have been reported from late 2003 to February in 2008. Avian influenza causes great economic damage due to high mortality and low egg production in birds, and, while not highly likely to cause infection, has high mortality in humans when infected. Since it is difficult to develop a fundamental avian influenza vaccine and has a very high spreading rate, it is necessary to minimize the spread of the disease and economic loss through early diagnoses.

As methods for diagnosing such a virus, immunological detection methods such as enzyme-linked immunosorbent assay (ELISA), enzyme immunoassay (EIA) and immunofluorescence assay (IFA) and an RNA detection method by RT-PCR are known, however, these methods are problematic in that it takes too much time to diagnose a virus and requires high cost for experiments or specificity and sensitivity are decreased due to non-specific reactions.

Therefore, it is necessary to develop a method for efficiently detecting a virus in a short period of time at low cost.

[Disclosure]

[Technical Problem]

The present invention is directed to providing a kit for detecting a virus, which can efficiently detect a virus within a short period of time, a method for detecting a virus, and a composition for detecting a virus.

[Technical Solution]

The present invention provides a kit for detecting a virus, which comprises a biomolecule that specifically reacts with a surface protein of a virus and a probe that reacts with the virus activated by the biomolecule, wherein the probe includes a marker bound with an amphiphilic polymer.

The present invention also provides a composition for detecting a virus, which comprises a biomolecule that specifically reacts with a surface protein of a virus and a probe that reacts with the virus activated by the biomolecule, wherein the probe includes a marker bound with an amphiphilic polymer.

The present invention also provides a method for detecting a virus, which comprises contacting a sample obtained from a subject with a biomolecule that specifically reacts with a surface protein of a virus and contacting the sample in contact with the biomolecule with a probe that reacts with the virus activated by the biomolecule, wherein the probe includes a marker bound with an amphiphilic polymer.

The present invention also provides a method for preparing a probe or a kit for detecting a virus, which includes binding an amphiphilic polymer with a marker.

[Advantageous Effects]

According to the present invention, a kit for detecting a virus can detect a virus at low cost with high efficiency in a short period of time, have higher sensitivity and accuracy than the conventional kit for detecting a virus by inhibiting initial release of a marker, and enhance preparation efficiency since it is not necessary to perform dialysis (cleaning) during preparation.

[Description of Drawings]

FIG. 1 is a diagram of the structure of a probe according to an embodiment of the present invention.
FIG. 2 is an image of a probe according to an embodiment of the present invention (scale bar: 100 nm).
FIG. 3 is an image of a probe according to an embodiment of the present invention (scale bar: 100 nm).
FIG. 4 is an image of a probe according to an embodiment of the present invention (scale bar: 100 nm).
FIG. 5 is a graph illustrating a comparison of average sizes of the probes according to embodiments of the present invention.

[Modes of the Invention]

All types of viruses including influenza virus and Ebola virus consist of a surface protein hemagglutinin (HA) binding to a host cell for invasion of host cells, neuraminidase involved in escape of mature virions from the host cells, an M2 ion channel for controlling balance in hydrogen ion concentration, ribonucleoprotein (RNP) containing the genetic information of a virus, etc. Based on the common characteristics of the viruses described above, the inventors have conducted studies to develop a method for detecting a virus at an early stage. As a result, they found that viruses can be detected by a simple method using a biomolecule that specifically reacts with a surface protein of a virus to activate viruses and a probe that reacts with the virus activated thereby to detect the existence of the viruses, and the present invention was completed.

Therefore, the present invention provides a kit for detecting a virus, which comprises a biomolecule that specifically reacts with a surface protein of a virus and a probe that reacts with the virus activated by the biomolecule, wherein the probe includes a marker bound with an amphiphilic polymer.

The probe of the present invention includes a "marker bound with an amphiphilic polymer." The "marker bound with an amphiphilic polymer" may be a "marker-binding amphiphilic polymer."

The term "probe" used herein refers to a material that reacts with a virus activated by a biomolecule that specifically reacts with a surface protein of a virus, thereby capable of detecting the presence of the virus or an active virus. The probe encompasses any material that has a considerably greater ability to react with the active virus compared to a material other than the virus or an inactive virus, or specifically reacts with the active virus, thereby is capable of significantly detecting the presence of the active virus, and the type and shape thereof are not specifically limited. In the specification, the probe is used in combination with a "detection factor."

In the case of the probe of the present invention including the "marker bound with an amphiphilic polymer," the marker is directly bound with the polymer which serves as a capsule or a carrier with the marker by forming a membrane, thereby it is possible to control initial release of the marker from the probe during detection. As a result of controlling initial release of the marker, it results in to enhance detection sensitivity and accuracy of probe, and to exhibit excellent preparation efficiency of the probe since step of dialysis (washing) during preparation of the probe may be skipped.

The term "binding" or "bonding" used herein refers to formation of a molecule with elements or ions. If the binding or bonding of elements or ions between the amphiphilic polymer and the marker, the present invention is not limited to the binding types, including a covalent bond, an ionic bond, a metallic bond and a coordinate bond.

For example, the binding may be an ester bond, an amide bond, an imine bond, a hydrazone bond or an acetal bond.

The term "marker" used herein refers to a material capable of detecting a reaction between a probe and an active virus. The type of the marker is not particularly limited and varies according to a change in the probe before and after the reaction, and any marker capable of confirming the changed property through a test may be used without limitation.

According to an embodiment of the present invention, the marker may further include a marker binding to an amphiphilic polymer (first marker) and a different type of marker (second marker).

The second marker may be bound with or carried in the polymer. That is, the probe of the present invention may further include the first marker bound with the amphiphilic polymer and the second marker carried in the probe, or a first marker-binding hydrophilic polymer and a second marker-binding hydrophilic polymer.

According to an embodiment of the present invention, the marker may include one or more selected from the group consisting of a self-quenched dye, a fluorescent dye, an electrochemiluminescent material, a quencher, a luminescent dye and a phosphorescent dye.

The markers binding to the amphiphilic polymer comprise one or more types.. Also, among the same types of markers, the markers used herein may be prepared by binding one or more different materials with each different amphiphilic polymer. In one embodiment, when the first marker is a fluorescent dye, the second marker may be a quencher, and in another embodiment, when the first marker is a quencher, the second marker may be a fluorescent dye.

According to an embodiment of the present invention, the probe may include a dye bound with an amphiphilic polymer and/or a quencher bound with an amphiphilic polymer.

In the present invention, the dye refers to a material selectively absorbing or emitting light with a specific wavelength. The light with a specific wavelength may be ultraviolet (UV) light, infrared (IR) light or visible light.

In the present invention, the self-quenched dye refers to a material which is quenched when adjacent to another and, when agglomerated dyes are released to be spread, emits fluorescence through dequenching.

In one embodiment, the self-quenched dye may be one or more selected from the group consisting of 3,3-dioctadecyloxacarbocyanine perchlorate (Dio; Dioc), 3,3 -dioctadecyl-5,5 - di(4-sulfophenyl)oxacarbocyanine sodium salt), 4-(4-(dihexadecylamino)styryl)-N-methylpyridinium iodide (DiA; 4-Di-16-ASP), 4-(4-(didecylamino)Styryl)-N-methylpyridinium iodide (4-Di-10-ASP), 1,1 -dioctadecyl-3,3,3,3 -tetramethylindocarbocyanine perchlorate (DiI), 1,1-dioctadecyl-6,6-di(4-sulfophenyl)-3,3,3,3-tetramethylindocarbocyanine, 4,4-diisothiocyanatostilbene-2,2-disulfonic acid disodium salt (DIDS), 1,1-dioctadecyl-3,3,3,3-tetramethylindotricarbocyanine iodide (DiR), 1,1-dioleyl-3,3,3,3-tetramethylindocarbocyanine, fluorescein, BODYPY, tetramethylrhodamine, octadecylodamine B chloride (R18), Alexa, Cyanine, and allopicocyanine. The fluorescent dye may have a mean diameter of 200 nm or less, or 50 to 10000 nm.

In one embodiment, the electrochemiluminescent material includes, but not limited to, tris(2,2'-bipyridyl)ruthenium(II) [Ru(bpy)32+].

In the present invention, the fluorescent dye is a material which absorbs light of a one wavelength, changes color, and reemits the light as fluorescence. The fluorescent dye is also called a luminescent dye, and any fluorescent dye well known in the art may be used without limitation. In one embodiment, the fluorescent dye may be one or more selected from the group consisting of Texas red, fluorescein, 4-chloro-7-nitrobenzofurazan (NBD-Cl), luminol, fullerene, and a compound with an aromatic group.

In one embodiment, the quencher refers to a material for removing excitation energy of a molecule, and inhibiting luminescence or fluorescence. The quencher is conventionally known in the art, and may be used without limitation to its type. According to an embodiment, the quencher may be one or more selected from the group consisting of BHQ-1, BHQ-2, and BHQ-3.

In one embodiment, a phosphorescent dye refers to a material exhibiting a photoluminescent effect by reemitting light with a wavelength changed from the absorbed wavelength of light of a light source. In one example, the phosphorescent dye may be, but is not limited to, a sulfide mineral ($X_mZ_n$, X is a metal element, and Z is a non-metal element) or a sulfide of an alkali earth metal.

The term "amphiphilic" used herein is also referred to as amphipathic, which means having both of hydrophilic and hydrophobic properties. Also, an amphiphilic particle refers to a particle having a hydrophilic domain and a hydrophobic domain. The amphiphilic polymer maybe selected from the group consisting of an A-B type amphiphilic block copolymer including a hydrophilic A block and a hydrophobic B block, a B-A-B type triblock copolymer, a lipid polymer and combinations thereof.

In one embodiment, the hydrophilic polymer may be one or more selected from the group consisting of polyalkyleneglycol (PAG), polyacrylic acid (PAA), polyacrylonitrile (PAN), polyethyleneoxide (PEO), polyvinylacetate (PVAc), polyethyleneglycol (PEG), polyvinylpyrrolidone, polyacrylamide, polyvinylalcohol (PVA) and hydrophilic poly(amino acid). For example, the hydrophilic polymer is preferably one or more selected from the group consisting of (mono)methoxypolyethylene glycol, (mono)acetoxypolyethylene glycol, polyethylene glycol, a copolymer of polyethylene and propyleneglycol, polyvinylpyrrolidone, poly(glutamine), polyglutamic acid, polythreonine, poly(asparagine), poly(arginine) and poly(serine). The hydrophilic A block may have a number average molecular weight at 200 to 50,000 daltons or 1,000 to 20,000 daltons.

In one embodiment, any hydrophobic polymer is possible used as long as it is a material capable of forming an amphiphilic polymer in combination with a hydrophilic polymer. In one embodiment, the hydrophobic B block may be one or more selected from the group consisting of polyester, poly(anhydride), hydrophobic poly(amino acid), polyorthoester and polyphosphazene. The hydrophobic B block is preferably one or more selected from the group consisting of polyleucine, polyisoleucine, polyvaline, polyphenylalanine, polyproline, polyglycine and polymethionine, polytryptophane, polyalanine, polylactide, polyglycolide, polycaprolactone, polydioxane-2-one, a copolymer of polylactide and glycolide, a copolymer of polylactide and dioxane-2-one, a copolymer of polylactide and caprolactone, and a copolymer of polyglycolide and caprolactone. The hydrophobic polymer also includes a derivative thereof. The hydrophobic B block has a number average molecular weight at 50 to 50,000 daltons or 200 to 20,000 daltons.

The term "lipid" or "lipid polymer" used herein encompasses phospholipids, lipid proteins, glycolipids, and cationic lipids as long as they are able to form a bilayer membrane structure, but the present invention is not limited thereto. Also, the lipid encompasses a naturally-induced lipid and a synthetic lipid derivative. The phospholipids include glycerophospholipids and phosphosphingolipids. The glycerophospholipids may include a diacrylglyceride structure and specifically include posphatidic acid (PA), lecithin (phosphatidylcholine, PC), cephalin and phosphoinositides. The cephalin phospholipids include phosphatidylserine (PS) and phosphatidylethanolamine (PE). Also, the phosphoinositide-like phospholipids include phosphatidylinositol (PI), phosphatidylinositol phosphate (PIP), phosphatidylinositol bisphosphate (PIP2) and phosphatidylinositol triphosphate (PIP3). The sphingophospholipids include ceramide phosphorylcholine (sphingomyelin, SPH), ceramide phosphorylethanolamine (sphingomyelin, Cer-PE) and ceramide phosphoryllipid.

There is no limit to the type of synthetic phospholipid derivative, but in one embodiment, the synthetic phospholipid derivative may be selected from the group consisting of 1,2-didodecanoyl-sn-glycero-3-ethylphosphocholine (EPC), 11,2-dioleoyl-sn-glycero-3-phosphocholine (DOPC), 1-palmitoyl-2-oleoyl-sn-glycero-3-phosphoethanolamine (POPE), 1-palmitoyl-2-oleoyl-sn-glycero-3-phospho-L-serine (POPS) and combinations thereof.

According to an embodiment of the present invention, the amphiphilic particle may be prepared by a known method without limitation. For example, the amphiphilic particle may be prepared by a method of dispersing an amphiphilic block copolymer including a hydrophilic domain and a hydrophobic domain in an aqueous solution and performing sonication, a method of dispersing or dissolving an amphiphilic block copolymer including a hydrophilic domain and a hydrophobic domain in an organic solvent and extracting or evaporating the organic solvent with an excess amount of water, a method of dialyzing an organic solvent with an excess amount of water after dispersion or dissolution of an amphiphilic block copolymer including a hydrophilic domain and a hydrophobic domain in an organic solvent, a method of dispersing or dissolving an amphiphilic block copolymer including a hydrophilic domain and a hydrophobic domain in an organic solvent and vigorously evaporating the solvent using a homogenizer or a high pressure emulsifier, thin film hydration, or the like.

The probe according to an embodiment of the present invention may be formed by self-assembling an amphiphilic block or polymer including a hydrophilic domain and a hydrophobic domain, resulting in forming a particle.

The probe of the present invention may have various structures or shapes according to the type of target virus or surface protein of a virus, the type of polymer used herein or preparation method.

According to an embodiment of the present invention, the probe may have a membrane structure.

The term "membrane structure" used herein refers to a structure in which the inner side thereof is surrounded by a membrane or shell. The membrane structure includes any type of encapsulation particles such as vesicles, artificial cells or microcapsules without limitation. In the specification, the membrane structure is used in combination with a shell structure. The membrane structure also includes a structure containing a fluid or a separate composition inside.

The membrane includes a mono layer or bilayer structure. The mono layer may be a membrane structure including "hydrophobic domain-hydrophilic domain," and in one embodiment, the membrane structure including a hydrophobic core and a hydrophilic shell in an aqueous solution. The bilayer is a membrane structure including "hydrophilic domain-hydrophobic domain-hydrophobic domain-hydrophilic domain." In one embodiment, the bilayer may include two mono layers which bind together. Also, the bilayer may be a structure including a hydrophilic core, a hydrophobic domain surrounding the hydrophilic core and a hydrophilic shell surrounding the hydrophobic domain.

The membrane structure includes both a single membrane and a multi membrane having two or more single membranes. In one example, when a particle having a single membrane is surrounded by one single membrane again, the particle may have a multi membrane. The single membrane and the multi membrane are concepts distinct from the mono layer and the bilayer. Specifically, a particle including only one membrane with a bilayer structure has a single membrane, and a particle in which a membrane with a mono layer structure is surrounded again by a membrane with a mono layer structure is referred to as a particle with a multi membrane structure.

A probe with the above-described membrane structure may be prepared by a conventional method to which the present invention belongs, for example, according to an embodiment, microfluidization using microfluidization equipment, high-pressure homogenization, emulsion or sonication, but the present invention is not limited thereto.

All types of particles with the above-described membrane structure may be included in the probe of the present invention without limitation. In one embodiment, particles may be selected from the group consisting of vesicles, micelles, polymersomes, colloidsomes, liposomes, droplets and combinations thereof, but the present invention is not limited thereto. The kit of the present invention may include one or more types of probes which have different membrane structures.

The term "micelle" refers to a particle having a hydrophobic core and a hydrophilic shell. In one embodiment, the micelle may be a micelle of a surfactant or a polymer self-assembled micelle. The micelle made by self assembly may have various shapes according to the type of polymer forming a copolymer, but the present invention is not limited thereto. The micelle also includes a multi membrane including two or more mono layers. The shape of the micelle may be, but not limited to, for example, a sphere, ellipsoid or cylinder. A probe with the micelle structure may further include components such as a marker and an inorganic particle in a hydrophobic core region.

The term "polymersome" refers to a particle which has a bilayer membrane structure with "hydrophilic domain-hydrophobic domain-hydrophobic domain-hydrophilic domain" and includes a polymer or copolymer in the hydrophobic and hydrophilic regions. The polymer or copolymer may include an artificially synthesized polymer or copolymer and a lipid, or an artificially synthesized polymer or copolymer. The artificially synthesized polymer or copolymer refers to any polymer or copolymer, not made of natural lipid. The polymersome includes a single membrane having a bilayer structure, or a multi membrane having two or more single membranes.

The polymersome may include an artificially synthesized polymer or copolymer, distinct from a liposome. The polymersome includes a single membrane having a bilayer structure or a multi membrane having two or more single membranes.

The term "liposome" refers to a particle having at least one lipid bilayer. The liposome may include both a single membrane and a multi membrane, as long as it has a lipid membrane mimicking an amphipathic biomembrane. A method for forming a liposome is well known in the art, and conventionally, a phospholipid may be obtained by suspension in a salted aqueous solution, or sonication with respect to an aqueous solution containing the phospholipid, but the present invention is not limited to the above-mentioned method.

The "colloidsome" refers to a colloidal particle with a size of 1 to 1000 nm or a construct in which the particles are densely packed. The colloidsome may include a mono layer and a bilayer according to types of polymers in hydrophilic and hydrophobic domains, which form a membrane. Also, the colloidsome may include both a single membrane and a multi membrane.

The "droplet" refers to a water drop-like particle in the membrane-structure particles. The droplet includes a mono layer and a bilayer, and both a single membrane and a multi membrane.

A probe of the present invention may be classified according to a shape, but the present invention is not limited thereto. In one embodiment, shapes of the probe include a rod, a sphere, a ring, a flat, a cylinder, an ellipsoid, a shape surrounded by a membrane and combinations thereof, but the present invention is not limited thereto.

The rod shape encompasses straight line shapes such as a stick, a bar, or a pole. The sphere encompasses round shapes including a complete sphere and an incomplete sphere. The ring shape generally means a spherical or round shape with a hollow core. The flat shape means a thin and even plate. According to an embodiment, a flat probe may be formed by placing a flat probe on or outside of a base material or carrier through coating.

In one embodiment of the present invention, when the mass fraction calculated according to the following equation is 0.25 to 0.40, the amphiphilic particle may be formed in a polymersome structure, and when the mass fraction is more than 0.40 and 0.70 or less, the amphiphilic particles have a micelle structure.

[Equation 1]

Mass Fraction= Mass of Hydrophilic Polymer/(Mass of Hydrophilic Polymer + Mass of

Hydrophobic Polymer)

Also, when the mass fraction is represented as mass % (percent), when the mass% satisfies 25% to 40%, the amphiphilic particles are formed in a polymersome structure, and when the mass% is more than 40% and 70% or less, the amphiphilic particles are formed in a micelle structure.

In one embodiment, the size of the amphiphilic particles is not limited. The probe may be nano or micro particles, which have a mean diameter of, for example, 50 to 10000 nm.

According to anther embodiment of the present invention, the probe may further include an inorganic particle selected from gold, silver and a combination thereof for more clearly determining whether the probe reacts with a virus.

A kit or composition of the present invention may include either one type or two or more types of probes.

The term "virus" used herein refers to an obligatory intracellular parasite, which has DNA or RNA as a nucleic acid, starts proliferating from the nucleic acid, does not proliferate through binary fission, and does not have an enzyme system required for ATP production.

The virus of the present invention encompasses a naked virus and an enveloped virus, and specifically, according to an embodiment of the present invention, the virus may be an enveloped virus.

Specifically, the enveloped virus encompasses DNA viruses such as herpesvirus, poxvirus and hepadnavirus, and RNA viruses such as flavivirus, togavirus, coronavirus, hepatitis D, orthomyxovirus, paramyxovirus, rhabdovirus, bunyavirus, filovirus and retrovirus.

The orthomyxovirus encompasses influenza virus A, influenza virus B, influenza virus C, isavirus, thogotovirus and quaranjavirus genera.

The coronavirus encompasses alpha coronavirus, beta coronavirus, gamma coronavirus and delta coronavirus genera.

The paramyxovirus encompasses paramyxovirus, rubella virus, morbillivirus and pneumovirus genera.

The virus includes a nucleic acid and a protein surrounding the nucleic acid. Such a viral protein is involved in protecting a viral genome, attaching and/or fusing virus particles to a cell, determines viral antigenicity, and includes a structural protein that maintains the shape of the virus and a non-structural protein that is associated with the synthesis of proteins and nucleic acids.

The term "surface protein of a virus" used herein refers to the structural protein. For example, the "surface protein" has specific antigenicity of a virus, a glycoprotein involved in attachment of a virus and fusion of an infected cell, and a fusion protein involved in fusion to a cell.

According to an embodiment of the present invention, the surface protein of the virus may be hemagglutinin (HA), spike protein or F protein.

According to an embodiment of the present invention, the virus of the present invention may include a surface protein that is attached to the cell membrane of a host cell, resulting in membrane fusion. Specifically, the virus of the present invention may include a surface protein with a property in which the surface protein present in an inactive form is transformed into an active form by a biomolecule to cause attachment and/or membrane fusion of a host cell to the cell membrane.

According to an embodiment of the present invention, the "virus" may be influenza virus or highly pathogenic influenza virus. The influenza virus includes a glycoprotein, hemagglutinin (HA) as a "surface protein" thereof. Hemagglutinin is expressed as one polypeptide (precursor HA0), and a cleavage site of $HA_0$ is divided into $HA_1$ and $HA_2$ through proteolysis, and in an acidic condition, a fusion peptide composed of hydrophobic amino acid residues is activated. HA has a different amino acid composition from the amino acids of the cleavage site due to pathogenicity of the influenza virus.

According to another embodiment of the present invention, the "virus" may be a coronavirus. The coronavirus includes spike protein as a surface protein, and the spike protein includes $S_1$ and $S_2$ portions. When the $S_1$ portion of the spike protein binds to a host cell, it is cleaved into $S_1$ and $S_2$ by a protease, and a hydrophobic domain at the end of $S_2$ is exposed and thus activated.

According to still another embodiment of the present invention, the "virus" may be a paramyxovirus. The paramyxovirus includes an HN protein as a surface protein. The HN protein is a binding (or attaching) protein that binds or attaches the virus to a host cell, and while it is present as a precursor $HN_0$ in the inactive state of the virus, it is activated by removing an amino acid residue from the C-terminus through hydrolysis. Also, a paramyxovirus includes F protein as a surface protein. While the F protein is present as a precursor $F_0$ in the inactive state of the virus, it is activated as a new form of the N-terminus $F_1$ after cleavage into $F_1$ and $F_2$ by a biomolecule. The F protein includes both a polybasic residue and a monobasic residue at the cleavage site. Preferably, the F protein may be an F protein that has a polybasic residue.

The term "reaction" used herein refers to changes in physical and/or chemical states such as condition, color, shape or chemical bond, when any material is in contact with a different material, or a phenomenon thereof. In one embodiment, when the surface protein of a virus reacts with a biomolecule, the surface protein of a virus is transformed, resulting in a state change to an activated virus such that a fusion peptide is formed. In another embodiment, the reaction between the activated virus and a probe may be fusion or aggregation. The fusion may be, according to an embodiment, a phenomenon in which the active virus binds to a part of the probe of the present invention. It may be understood that the fusion also includes a phenomenon in which a substance in the probe is released to the outside through fusion. In one example, the active virus may be detected by confirming whether the active virus is fused to the probe using a mechanism in which a fusion peptide present in the surface protein of the active virus induces fusion of an endosome to the cell membrane. The aggregation refers to a phenomenon in which molecules or particles bind together in a solution. In one embodiment of the present invention, the aggregation refers to a phenomenon in which probes are coagulated by reducing the stability of the probe of the present invention due to a virus which is a detection target, resulting in coagulation and/or precipitation.

The term "biomolecule" used herein refers to a molecule required for the structure, function or signal transduction in a living organism. The biomolecule may include a specific structure and/or region for a function or signal transduction. The specific structure for such a biomolecule is a driving force generating a reaction, through an intermolecular binding reaction. The biomolecule includes an amino acid and a protein, a sugar and a carbohydrate, a fatty acid and a lipid, and a nucleotide and a nucleic acid, and encompasses all products made in a living organism and/or artificially synthesized products. According to an embodiment of the present invention, the "biomolecule" may be an enzyme, which is a protein consisting of approximately 62 to 2500 amino acid residues.

In one embodiment, the enzyme may be a protease. In one example, the first synthesized form of the enzyme is inactivated, but in a protein exhibiting activity due to removing(or cleaving) a specific portion, the protease cleaves the specific portion, resulting in activation of the inactivated protein. The protease may be any enzyme capable of cleaving a specific portion without limitation to its type.

In one embodiment, the protease may be one or more selected from the group consisting of furin, trypsin, serine, endoprotease and carboxypeptidase. The protease encompasses derivatives or modifications thereof. For example, the protease may include recombinant human furin, recombinant mouse furin, trypsin-EDTA, acetylated trypsin, N-tosyl-L-phenylalanine chloromethyl ketone-trypsin (TPCK-trypsin), trypsin-acrylic, furin-like protease, Kex2 protease, transmembrane protease serine, TMPRSS2, TMPRSS4, tryptase clara, plasmin, serine protease, subtilisin-like endoprotease, carboxypeptidase and combinations thereof.

In one embodiment, when trypsin reacts with the surface protein of influenza virus, hemagglutinin (HA), enzymatic degradation of the trypsin occurs at His57-Asp102-Ser195, and enzymatic degradation of furin occurs at His194-Asp153-Ser368. Since trypsin degrades both the hemagglutinin of highly and lowly pathogenic influenza virus, the highly/lowlydly pathogenic influenza virus may be detected using trypsin. In addition, since furin may degrade only hemagglutinin of a highly pathogenic influenza virus, the highly pathogenic influenza virus may be detected using furin.

In another embodiment, when the protease reacts with the surface protein of the coronavirus, hydrolysis occurs at the boundary between the S1 and S2 regions. Therefore, as the hydrophobic domain of S2 is exposed by cleavage through the hydrolysis, the coronavirus is activated for detection using a probe of the present invention.

In yet another embodiment, a biomolecule reacting with the surface protein of a paramyxovirus may be a protease or carboxylpeptidase, which facilitates cleavage at an arginine residue of the C-terminus (carboxyl side). The protease may recognize and cleave F protein containing an R-X-K/R-R arrangement. As a specific example, the protease may be furin or subtilisin-like endoprotease.

The term "active virus" or "activated virus" used herein refers to a virus in which a surface protein thereof is activated by a biomolecule that specifically reacts with the surface protein of the virus. Here, the activation of the surface protein of a virus means that the surface protein of the virus is changed to be ready for a reaction between a host cell and/or a probe, for example, a specific reaction between a host cell and/or a probe. Here, the term "activation of a virus" refers to a process of converting an inactive virus into an active virus. Since the activation of a virus is necessary for infection by a virus, the viral infection may be determined by detecting the presence of the active virus.

In one embodiment, activation of the surface protein of a virus means that a state in which a fusion protein or fusion peptide that is present on the surface protein of the virus is able to fuse with the surface of the membrane of a host cell or the surface of a probe of the present invention.

In one embodiment, for the influenza virus, activated virus means conversion of the surface protein hemagglutinin (HA) that exposes the fusion peptide present in the hemagglutinin in an inactivated state through degradation by an enzyme.

That is, an enzyme for specifically degrading the cleavage site of HA includes furin and/or trypsin. Since furin may degrade only hemagglutinin of a highly pathogenic influenza virus, when HA of the influenza virus is degraded by furin, the influenza virus exhibits high pathogenicity. Also, since trypsin may degrade both hemagglutinin of the highly and lowly pathogenic influenza virus, when the hemagglutinin is degraded by trypsin, the influenza virus exhibits high or low pathogenicity. A restriction site of the lowly pathogenic influenza virus includes -R- in the C-terminus, and a restriction site of the highly pathogenic influenza virus consists of R/K-R-K-K-R.

When HA is activated by an enzyme specifically degrading the restriction site of HA, rearrangement of HA occurs, and thus the fusion peptide in HA is exposed to the outside, thereby resulting in an active virus. Likewise, when the influenza virus activated under an acidic condition encounters a probe according to an embodiment of the present invention, the reaction between the influenza virus and the probe occurs due to the fusion peptide of the influenza virus. By measuring signal varying with the probe reaction, a viral infection may be detected.

In another embodiment, for a coronavirus, an activated virus means that a surface protein, i.e., spike protein (S protein), is changed such that a hydrophobic domain present in the spike protein in an inactivated form is exposed due to a biomolecule. Specifically, when an S1 region of the spike protein binds to a host cell, the spike protein is cleaved into $S_1$ and $S_2$ by a protease, and a hydrophobic domain at the end of S2 is exposed, resulting in activation of the virus. The exposed hydrophobic domain of S2 is attached to a hydrophobic domain in the cell membrane of the host cell, and on the other side, membrane fusion takes place due to a conformational change in which the middle part of the S2 region is folded while bound to the outer membrane of the virus. Therefore, when the probe of the present invention reacts with the activated coronavirus, the probe's stability is changed, various signals are generated, and thus a viral infection may be detected by measuring such a change.

In another embodiment, a virus activated in a paramyxovirus refers to a state in which a residue of the C-terminus is removed from a HN protein by a biomolecule. Specifically, while the HN protein retains an inactivated state ($HN_0$), when approximately 90 residues of the C-terminus are removed, the HN protein is activated to allow the paramyxovirus to be attached to the membrane of a host cell or target. Further, active paramyxovirus means that F protein is cleaved into $F_1$ and $F_2$ by a biomolecule. In the inactive paramyxovirus, F protein binds to a biomolecule in a precursor $F_0$ state, the F protein is cleaved into $F_1$ and $F_2$, thereby becoming an active virus capable of fusing with the cell membrane of a host cell. That is, membrane fusion is initiated with formation of an active surface protein containing SS-bonding chain $F_1$ and $F_2$, and fixation of an *N*-terminus residue of $F_1$ exhibiting broad hydrophobicity to the cell membrane of a host cell or a target. Therefore, the paramyxovirus is activated by reacting a biomolecule of the present invention with the surface protein, and the activated virus reacts with the probe of the present invention again, resulting in a change in the probe. Accordingly, the virus may be detected by measuring the change.

In one embodiment of the present invention, the kit or composition may further include an acidic material with pH 6 or less. For example, the acidic material with pH 6 or less may be an acid solution, and the acid solution with the above range of pH may be included in the kit and may be directly prepared for use. The acid solution with pH 6 or less may be any solution satisfying the pH condition without limitation. As the acid solution, a stock solution may be commercially available or directly prepared for use. For example, a thick acid solution, a stock solution, may be diluted in water to have pH 6 or less. For example, the acid solution may have pH 4 to 6 or pH 5 to 5.7. Since the above-mentioned range is similar to the pH range in a host cell, detection efficiency may be increased.

In one embodiment, the kit and composition of the present invention may further include an adjuvant.

The adjuvant may be used for a biomolecule to help the reaction with a virus or may help the reaction of a target molecule with a probe. As a specific example, the adjuvant may be used to help the reaction of a protease with a surface protein or to help the reaction of a target molecule including the activated surface protein of an active virus and the antigenic protein of the active virus with a probe.

The term "adjuvant" used herein may be any material that is able to increase detection sensitivity and/or specificity for a virus through the above-described reaction. The detection sensitivity and/or specificity of a virus may be increased by increasing the rate of the reaction, decreasing the minimum value at which the reaction takes place, or inhibiting other reactions except the reaction between a surface protein and a biomolecule, but the present invention is not limited to the above-mentioned mechanisms. The adjuvant encompasses all materials that are able to increase detection sensitivity and/or specificity according to types of biomolecules and surface proteins reacting therewith without limitation.

A specific example of the adjuvant may be a ketone.

The ketone compound may encompass, for example, phenylethylchloromethylketone, tosyl phenylalanyl chloromethyl ketone (TPCK) and combinations thereof, but the present invention is not limited thereto.

In one embodiment of the present invention, for the detection of influenza virus, a ketone may further be included as an adjuvant. In this case, the sensitivity of the kit may be further increased by reducing the activities of other enzymes and increasing the activity of a hemagglutinin (HA) protease, particularly, trypsin.

The present invention relates to a method for detecting a virus.

The detection method of the present invention includes contacting a sample with a biomolecule, and contacting the sample in contact with the biomolecule with a probe. Specifically, the detection method of the present invention includes contacting a sample obtained from a subject with a biomolecule that specifically reacts with a surface protein of a virus; and contacting the sample in contact with the biomolecule with a probe that reacts with the virus activated by the biomolecule, wherein the probe includes a marker bound with an amphiphilic polymer.

The detection method of the present invention may detect a virus by confirming the presence or absence of a reaction occurring due to such contacts. As an example, an active virus may be detected by examining whether a probe is fused with an active virus using the mechanism in which the cell membrane of an endosome is fused by a fusion peptide present in the surface protein of the active virus. In another example, an active virus may be detected by examining whether a probe is aggregated using the mechanism in which stability of the probe is reduced by a fusion peptide present in the surface protein of the active virus.

By a method for examining the reaction in the present invention, a signal or change of the probe generated by the reaction may be detected. As an example, the change or signal generated when the active virus reacts with the probe may be a change in one or more selected from the group consisting of fluorescence intensity, luminescence intensity, phosphorescence intensity, absorbance, an electrical signal, a signal from surface-enhanced Raman spectroscopy (SERS), a field-effect transistor (FET), a color and the dispersity of probes, but the present invention is not limited thereto.

For example, when a self-quenched dye is used as a marker capable of determining the reaction of a probe, the marker such as the self-quenched dye binding to a polymer of the probe is released or exposed to the outside of the probe due to the reaction of the active virus with the probe. The probe from which the marker is exposed or released emits fluorescence due to dequenching. Therefore, the infection by a virus may be detected by measuring the change in fluorescence intensity.

Also, when a fluorescent dye is used as a marker for determining the reaction of a probe, following the reaction of the fluorescent dye with the probe by an active virus, the fluorescent dye binding to a polymer of the probe may be exposed to the outside of a membrane, or the fluorescent dye carried in the probe may be eluted to the outside of the polymer membrane due to disintegration of the polymer membrane of the probe particle, and then the fluorescence intensity may be measured to detect infection by a virus. Since luminescence intensity, phosphorescence intensity and absorbance intensity vary according to wavelength, like the fluorescence intensity, the infection by a virus may be detected by measuring the intensities. Particularly, among such fluorescent dyes, luminol or fullerene may be used to visually detect a change in color without measuring fluorescence intensity using a special apparatus.

Also, when one probe includes a fluorescent dye and a quencher as markers, following the reaction between an active virus and the probe, if a polymer membrane is not disintegrated, the quencher and the fluorescent dye are present in the membrane, and thus fluorescence may not be detected, but if the dye is exposed or eluted due to membrane disintegration, fluorescence intensity of the probe is changed, and thus the presence of a virus may be detected by measuring such a change.

In addition to the fluorescent dye, an electrochemiluminescent material such as tris(2,2'-bipyridyl)ruthenium(II)[Ru(bpy)32+]) may be included, and in this case, a change in color may be visually observed.

Also, a virus may be detected by measuring an electrical signal using a nano gap sensor. The electrical signal may be measured as a current change or by using an FET. The nano gap sensor refers to a sensor including an electrode having a gap interval of approximately 100 nm or less. For example, when a fluorescent dye is used as a marker capable of determining the reaction of a probe, the fluorescent dye in the probe is released due to fusion of an active virus with the probe and placed in the nano gap of the nano gap sensor. Here, the viral infection may be detected by measuring a current change of a metal particle such as gold, silver, chromium, titanium, platinum, copper, palladium, indium tin oxide (ITO) or aluminum, which has been placed in the nano gap sensor in advance. The metal particle may have a mean diameter of a few nanometers, for example, 2 to 4 nm.

Further, a virus may be detected by measuring an SERS signal. To measure the SERS signal, a base material capable of binding to a sample may be used. The base material may be a nanoparticle, a colloid or a liquid, but the present invention is not limited thereto. For example, when a fluorescent dye is used as a marker capable of determining fusion of a probe, due to the reaction of an active virus with the probe, the fluorescent dye in the probe is released. Following binding of the release fluorescent dye with the base material, a metal particle is introduced again to the fluorescent dye bound with the base material. Raman spectroscopy may be amplified by introducing the metal particle such as gold or silver. Afterward, the viral infection may be detected by measuring the Raman spectrum of the fluorescent dye using a Raman spectrometer.

As another example, a virus may be detected by confirming aggregation, that is, a change in dispersity of probes due to the aggregation of the probes by an active virus. For example, when the probe includes inorganic particles, the particles are aggregated due to reduction of stability of the probe by the active virus.

In one embodiment, at least one of the procedures may be performed under the condition of pH at 6 or less. For example, at least one of the procedures may be performed under an acidic condition such as pH 4 to pH 6 or pH 5 to pH 5.5. In one embodiment of the present invention, detection of influenza virus in an acidic condition may provide a condition very similar to the environment in a host cell, and thus detection accuracy may be increased.

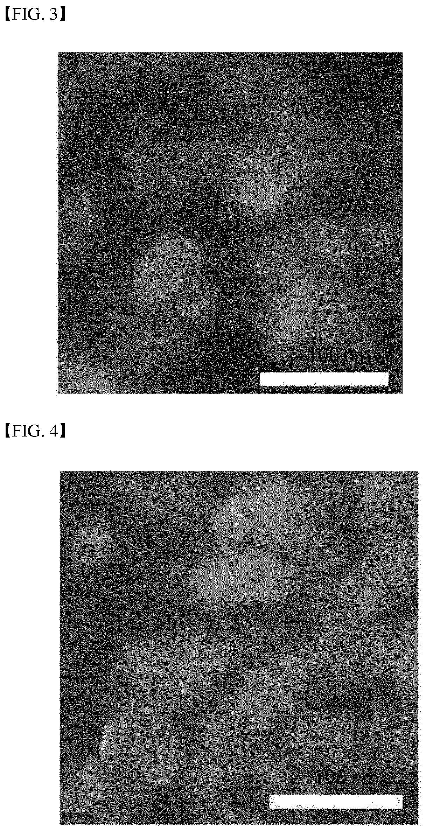

FIG. 3 shows a schematic diagram illustrating a method for detecting influenza virus according to an embodiment of the present invention. Simply, under the condition of pH 6 or less, pH 4 to pH 6, or pH 5 to pH 5.5, a sample obtained from a subject is added to a well in which furin and/or trypsin are present to activate influenza virus present in the sample, and then treated with a probe according to an embodiment of the present invention. When influenza virus is present, a reaction between active influenza virus and a probe occurs due to a fusion peptide, and the presence of the influenza virus may be detected by measuring signals varying according to the reaction. As described above, the reaction between the active influenza virus and the probe may be fusion or aggregation. The reaction between the active influenza virus and the probe and a signal varying thereby are described above.

The term "sample" used herein refers to a material obtained to represent a parent for detection of a virus. In one embodiment, the sample may be a saliva, oral mucus or excreta sample.

The present invention also relates to a composition for detecting a virus, which includes a biomolecule that specifically reacts with a surface protein of a virus and a probe that reacts with the virus activated the biomolecule. Here, all of the details about the kit for detecting a virus and the method for detecting a virus may be applied to the composition for detecting a virus.

The present invention also relates to a method for preparing a probe or kit for detecting a virus, which includes binding an amphiphilic polymer with a marker.

According to an embodiment, the method includes binding an amphiphilic polymer with a first marker and forming particles in a membrane structure using the marker bound with the amphiphilic polymer.

According to another embodiment, before the particle forming step, the method may further include dispersing the first marker-binding amphiphilic polymer and a second marker in a solvent. The second marker-carrying probe may be prepared by forming particles in a membrane structure from the solution in which the second marker is also dispersed.

According to still another embodiment, the method may include binding an amphiphilic polymer with a first marker, binding an amphiphilic polymer with a second marker, and forming particles in a membrane structure by dispersing the first marker-binding amphiphilic polymer and the second marker-binding amphiphilic polymer in a solvent.

The forming of the particles in a membrane structure may be performed by a method for forming a colloid, liposome, micelle, or polymersome, which is conventionally used in the present invention, and, for example, particles of the membrane structure may be prepared by a method for dispersing an amphiphilic block copolymer including a hydrophilic domain and a hydrophobic domain in an aqueous solution and performing sonication, a method for dispersing or dissolving an amphiphilic block copolymer including a hydrophilic domain and a hydrophobic domain in an organic solvent and extracting or evaporating the organic solvent with an excess amount of water, a method for dialyzing an organic solvent with an excess amount of water after dispersion or dissolution of an amphiphilic block copolymer including a hydrophilic domain and a hydrophobic domain, a method for dispersing or dissolving an amphiphilic block copolymer including a hydrophilic domain and a hydrophobic domain in an organic solvent and vigorously evaporating the solvent using a homogenizer or a high pressure emulsifier, or thin film hydration.

In the formation of particles in a membrane structure, the amphiphilic polymer not binding to a marker and the marker-binding amphiphilic polymer may be mixed at a weight ratio of 1 : 1 to 10.

The probe according to the preparation method of the present invention may form a membrane such that the polymer serving as a capsule or carrier directly binds to the marker. Therefore, release of the marker in the early stage of the detection may be controlled, and thereby, it is not necessary to perform dialysis during the preparation, resulting in obtaining excellent preparation efficiency for the kit for detecting a virus.

Here, all of the details about the kit for detecting a virus and the method for detecting a virus may be applied to the method for preparing a probe for detecting a virus.

Hereinafter, the present invention will be described with reference to examples. The following examples are merely provided to exemplify the present invention, and the scope of the present invention is not limited thereto.

**[Examples]**

**[Preparation Example 1] Preparation of probe particles comprising hydrophilic polymer-hydrophobic polymer-fluorescent dye conjugates and quencher on the inside of** the probe

Micelle- or polymersome-type probes in which a quencher is comprised were prepared using an amphiphilic copolymer to which a fluorescent dye binds as a marker. mPEG-NH$_2$ was purchased from Laysan Bio, Inc., Cy5.5-NHS ester was purchased from GE Healthcare, BHQ3-NHS ester was purchased from Biosearch Technologies, and 1-ethyl-3-(3-dimethylaminopropyl)carbodiimide and dimethylsulfoxide were purchased from Sigma Aldrich. Specifically, 2.5 mg of Cy5.5-NHS ester and 8.9 mg of 1-ethyl-3-(3-dimethylaminopropyl)carbodiimide were added to 10 mg of mPEG-b-pLeu and dissolved in 2 mL dimethylsulfoxide to carry out a reaction at room temperature for 12 hours. The reaction-completed solution was precipitated in 4 mL cold ethyl ether, and centrifuged to remove a supernatant. After lyophilization, a hydrophilic polymer-hydrophobic polymer-fluorescent dye conjugate was obtained.

Probes were prepared by a method of forming a particle by preparing 10 mg of a 1:1 mixture of mPEG-b-pLeu and the mPEG-b-pLeu-fluorescent dye conjugate, mixing 0.04 mg of BHQ3-NHS ester, and performing dialysis. After dialysis, a cleaning step was omitted.

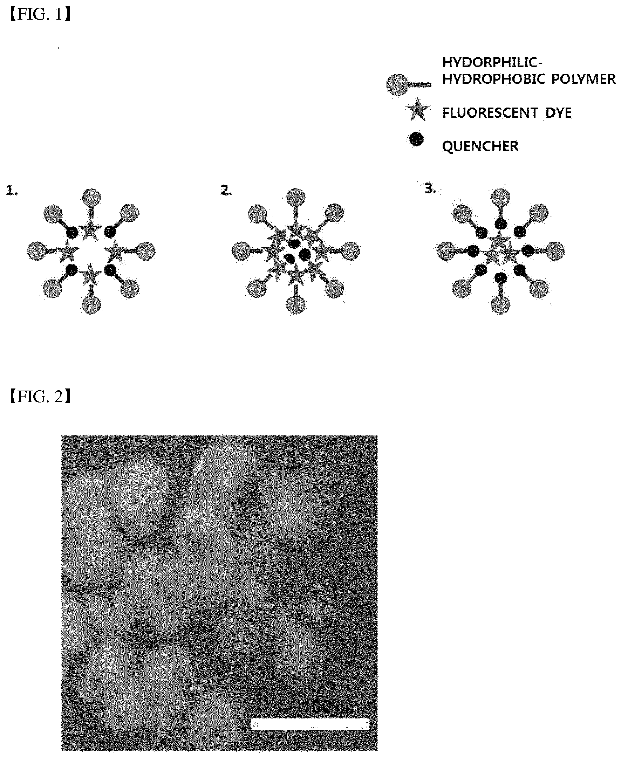

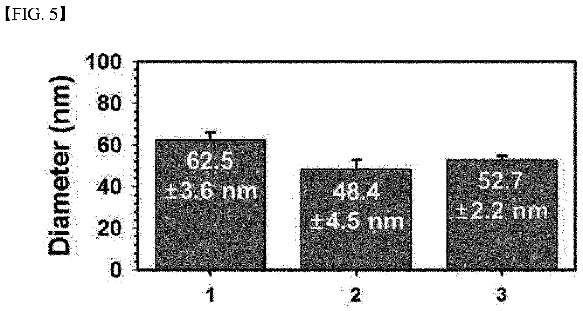

The morphology of the prepared probes was visualized by a transmission electron microscope (TEM), which is shown in FIG. 2, and the sizes of the prepared probe particles were measured using dynamic light scattering (DLS). The mean diameters measured thereby are shown in FIG. 5.

As shown in FIGS. 2 and 5, it was confirmed that the probes were prepared in either an incomplete spherical or ellipsoidal type to have a mean diameter of 62.5±3.6 nm.

Moreover, among such probes, the polymersome-type probe had a molecular weight of 6500 g/mol, and the micelle-type probe had a molecular weight of 3300 g/mol.

**[Preparation Example 2] Preparation of probe particles comprising hydrophilic polymer-hydrophobic polymer-quencher conjugates and fluorescent dyes on the inside of the probe**

Probes were prepared by the same method as described in Preparation Example 1, except that a quencher was bound to an amphiphilic polymer and a fluorescent dye was carried.

mPEG-NH$_2$ was purchased from Laysan Bio, Inc., Cy5.5-NHS ester was purchased from GE Healthcare, BHQ3-NHS ester was purchased from Biosearch Technologies, and 1-ethyl-3-(3-dimethylaminopropyl)carbodiimide and dimethylsulfoxide were purchased from Sigma Aldrich.

Specifically, 10 mg mPEG-b-pLeu, 1.8 mg BHQ3-NHS ester and 8.9 mg 1-ethyl-3-(3-dimethylaminopropyl)carbodiimide were dissolved in 2 mL dimethylsulfoxide to carry out a reaction at room temperature for 12 hours. The reaction-completed solution was precipitated in 4 mL cold ethyl ether and centrifuged to remove a supernatant. After lyophilization, a hydrophilic polymer-hydrophobic polymer-quencher conjugate was obtained.

Probes were prepared by a method of forming a particle by preparing 10 mg of a 1:1 mixture of mPEG-b-pLeu and the mPEG-b-pLeu-quencher conjugate, mixing 0.04 mg of cy5.5-NHS ester, and performing dialysis.

The morphology of the prepared probes was visualized by TEM, which is shown in FIG. 3, and the sizes of the prepared probe particles were measured using DLS. The mean diameters measured thereby are shown in FIG. 5.

As shown in FIGS. 3 and 5, it was confirmed that the probes were prepared in either an incomplete spherical or ellipsoidal type and to have a mean diameter of 48.4±4.5 nm.

**[Preparation Example 3] Preparation of probes including hydrophilic polymer-hydrophobic polymer-quencher conjugates and hydrophilic polymer-hydrophobic polymer-fluorescent dye conjugates**

Probes were prepared by the same method as described in Preparation Example 1, except that a fluorescent dye-binding polymer and a quencher-binding polymer were used.

mPEG-NH$_2$ was purchased from Laysan Bio, Inc., Cy5.5-NHS ester was purchased from GE Healthcare, BHQ3-NHS ester was purchased from Biosearch Technologies, and 1-ethyl-3-(3-dimethylaminopropyl)carbodiimide and dimethylsulfoxide were purchased from Sigma Aldrich.

Specifically, 2.5 mg of Cy5.5-NHS ester and 8.9mg of 1-ethyl-3-(3-dimethylaminopropyl)carbodiimide were added to 10 mg of mPEG-b-pLeu and dissolved in 2 mL of dimethylsulfoxide to carry out a reaction at room temperature for 12 hours. The reaction-completed solution was precipitated in 4 mL cold ethyl ether and centrifuged to remove a supernatant. After lyophilization, a hydrophilic polymer-hydrophobic polymer-fluorescent dye conjugate was obtained.

1.8 mg of BHQ3-NHS ester and 8.9 mg of 1-ethyl-3-(3-dimethylaminopropyl)carbodiimide were added to 10 mg of mPEG-b-pLeu and dissolved in 2 mL of dimethylsulfoxide to carry out a reaction at room temperature for 12 hours. The reaction-completed solution was precipitated in 4 mL cold ethyl ether and centrifuged to remove a supernatant. After lyophilization, a hydrophilic polymer-hydrophobic polymer-quencher conjugate was obtained.

Amphiphilic particle probes were prepared by preparing 10 mg of a 1:1 mixture of the obtained conjugates and performing dialysis.

The morphology of the prepared probes was visualized by TEM, which is shown in FIG. 4, and the sizes of the prepared probe particles were measured using DLS. The mean diameters measured thereby are shown in FIG. 5.

As shown in FIGS. 4 and 5, it was confirmed that the probes were prepared in either an incomplete spherical or ellipsoidal type and to have a mean diameter of $52.7 \pm 2.2$ nm.

**[Experimental Example 1] Confirmation of virus detection effect of probe**

A virus detection effect of the probe prepared by the method of Preparation Example 1 was confirmed.

Specifically, experiments were performed on target viruses, for example, three types of highly pathogenic influenza viruses such as H5N1_01, H5N1_02 and H5N6 and eleven types of lowly pathogenic influenza viruses such as H1N1_01, H1N1_02, H2N1, H2N4, H3N8, H5N2, H5N3, H7N9, H7N7, H9N2_01 and H9N2_02. As biomolecules reacting with viruses, furin and trypsin, which are capable of specifically degrading the surface protein of influenza virus, which is hemagglutinin, were used. lowly pathogenic influenza viruses were provided from Green Cross Veterinary Product Co., Ltd., and highly pathogenic viruses were provided from Hanoi University of agriculture. For a pH condition, a proper pH was achieved using an acidic stock solution.

Enzymes suitable for individual experimental groups were added to each well of a 96-well plate, and the enzyme-contained wells were inoculated with each type of target viruses. Subsequently, the resulting well was inoculated with the probe of the present invention, and then a change in fluorescence intensity of the probe was measured (Ex: 675 nm, Em: 694 nm).

For the total 14 types of influenza viruses, detection environments (pH conditions of the wells) were divided into pH 5.5 and pH 7.4, and the experiment was performed for each of 1) a furin only-treated group, 2) a trypsin only-treated group, 3) a furin and trypsin-treated group, 4) a furin and excreta-treated group, 5) trypsin and excreta-treated group and 6) excreta only-treated group (enzyme-free) according to each pH condition. The results are shown in Tables 1 and 2.

[Table 1]

| Sample | pH 5.5 | | pH 7.4 | | Furin + Trypsin | | Enzyme-free | |
|---|---|---|---|---|---|---|---|---|
| | Furin | Trypsin | Furin | Trypsin | pH 5.5 | pH 7.4 | pH 5.5 | pH 7.4 |
| H5N1_01 | 2841 | 2952 | 348 | 483 | 2521 | 371 | 540 | 497 |
| H5N1_02 | 2133 | 2958 | 316 | 594 | 2858 | 573 | 469 | 451 |
| H5N6 | 2415 | 2744 | 389 | 501 | 2384 | 408 | 406 | 544 |
| H1N1_01 | 492 | 3353 | 316 | 573 | 4316 | 550 | 303 | 419 |
| H1N1_02 | 594 | 4548 | 301 | 499 | 4429 | 409 | 371 | 374 |
| H2N1 | 371 | 2213 | 306 | 552 | 2877 | 520 | 564 | 378 |
| H2N4 | 426 | 4025 | 333 | 472 | 4800 | 427 | 457 | 585 |
| H3N8 | 399 | 3600 | 423 | 348 | 3370 | 413 | 440 | 317 |
| H5N2 | 429 | 2369 | 589 | 378 | 2124 | 473 | 427 | 576 |
| H5N3 | 482 | 3252 | 595 | 587 | 4455 | 367 | 427 | 571 |
| H7N9 | 545 | 3992 | 341 | 329 | 4045 | 572 | 391 | 397 |

(continued)

| Sample | pH 5.5 | | pH 7.4 | | Furin + Trypsin | | Enzyme-free | |
|---|---|---|---|---|---|---|---|---|
| | Furin | Trypsin | Furin | Trypsin | pH 5.5 | pH 7.4 | pH 5.5 | pH 7.4 |
| H7N7 | 432 | 2605 | 308 | 434 | 2606 | 524 | 593 | 442 |
| H9N2_01 | 353 | 3754 | 332 | 576 | 3534 | 451 | 451 | 382 |
| H9N2_02 | 418 | 3282 | 551 | 370 | 3520 | 346 | 571 | 337 |

[Table 2]

| Sample | Enzyme + Excreta (pH 5.5) | | Excreta-treated group (enzyme-free) | |
|---|---|---|---|---|
| | Furin | Trypsin | pH 5.5 | pH 7.4 |
| H5N1_01 | 2444 | 2662 | 358 | 477 |
| H5N1_02 | 2281 | 2879 | 596 | 480 |
| H5N6 | 2833 | 2732 | 419 | 551 |
| H1N1_01 | 4152 | 3604 | 330 | 339 |
| H1N1_02 | 4218 | 3701 | 399 | 340 |
| H2N1 | 2285 | 2934 | 571 | 426 |
| H2N4 | 3334 | 4147 | 354 | 565 |
| H3N8 | 3050 | 4373 | 356 | 422 |
| H5N2 | 2676 | 2927 | 487 | 429 |
| H5N3 | 4043 | 3217 | 420 | 578 |
| H7N9 | 4146 | 3871 | 358 | 357 |
| H7N7 | 2632 | 2931 | 546 | 323 |
| H9N2_01 | 3117 | 4842 | 433 | 386 |
| H9N2_02 | 3748 | 4485 | 590 | 504 |

As shown in Tables 1 and 2, in the enzyme-free group and the non-acidic groups, no fluorescence change of the probe was detected. However, in the highly pathogenic influenza virus groups, when the acidic condition is satisfied, when both the probe and trypsin were treated, all of the highly/lowly pathogenic influenza virus groups showed fluorescence changes, and when furin was treated, only the highly pathogenic influenza virus group showed a fluorescence change. It was seen that trypsin can be used to detect both highly/lowly pathogenic influenza viruses, and furin can be used to detect only lowly pathogenic influenza viruses. Also, although the enzyme and excreta were treated together, when the acidic condition and enzyme are present, a fluorescent change was detected, which indicates that the detection ability can be retained even when combined with different materials.

**[Experimental Example 2] Confirmation of virus detection effect of probe**

A virus detection effect of the probe prepared by the method of Preparation Example 2 was confirmed.
Specifically, the experiment was performed under the same conditions as described in Experimental Example 1, except that the probe of Preparation Example 2 was used, instead of the probe of Preparation Example 1. Results of the measurement of fluorescence intensity are shown in Tables 3 and 4.

[Table 3]

| Sample | pH 5.5 | | pH 7.4 | | Furin + Trypsin | | Enzyme-free | |
|---|---|---|---|---|---|---|---|---|
| | Furin | Trypsin | Furin | Trypsin | pH 5.5 | pH 7.4 | pH 5.5 | pH 7.4 |
| H5N1_01 | 4682 | 4001 | 459 | 557 | 4445 | 469 | 547 | 361 |
| H5N1_02 | 4837 | 4544 | 480 | 588 | 4261 | 310 | 440 | 404 |
| H5N6 | 2719 | 2761 | 399 | 302 | 2203 | 330 | 386 | 422 |
| H1N1_01 | 374 | 4023 | 560 | 371 | 3632 | 353 | 513 | 505 |
| H1N1_02 | 364 | 4719 | 306 | 529 | 4072 | 314 | 540 | 579 |
| H2N1 | 333 | 2117 | 397 | 364 | 2788 | 479 | 353 | 473 |
| H2N4 | 347 | 3090 | 377 | 400 | 4530 | 443 | 582 | 363 |
| H3N8 | 442 | 4072 | 552 | 446 | 4537 | 479 | 507 | 442 |
| H5N2 | 323 | 2340 | 438 | 388 | 2639 | 425 | 380 | 477 |
| H5N3 | 590 | 4733 | 357 | 310 | 3136 | 395 | 447 | 584 |
| H7N9 | 461 | 4599 | 537 | 562 | 4529 | 414 | 303 | 539 |
| H7N7 | 401 | 2067 | 481 | 330 | 2187 | 330 | 389 | 595 |
| H9N2_01 | 443 | 4577 | 591 | 365 | 4767 | 404 | 327 | 407 |
| H9N2_02 | 571 | 4716 | 359 | 566 | 4144 | 386 | 533 | 354 |

[Table 4]

| Sample | Enzyme + excreta (pH 5.5) | | Excreta-treated group (Enzyme-free) | |
|---|---|---|---|---|
| | Furin | Trypsin | pH 5.5 | pH 7.4 |
| H5N1_01 | 3762 | 4226 | 345 | 572 |
| H5N1_02 | 3442 | 4806 | 523 | 545 |
| H5N6 | 2821 | 2031 | 318 | 377 |
| H1N1_01 | 4923 | 3062 | 449 | 507 |
| H1N1_02 | 4376 | 4432 | 353 | 521 |
| H2N1 | 2672 | 2526 | 550 | 396 |
| H2N4 | 4433 | 3280 | 579 | 403 |
| H3N8 | 4992 | 4650 | 350 | 337 |
| H5N2 | 2104 | 2791 | 554 | 338 |
| H5N3 | 4068 | 3311 | 541 | 318 |
| H7N9 | 3216 | 4080 | 420 | 467 |
| H7N7 | 2237 | 2287 | 373 | 375 |
| H9N2_01 | 4322 | 3557 | 419 | 481 |
| H9N2_02 | 4266 | 3219 | 339 | 423 |

As shown in Tables 3 and 4, when furin was treated, the enzyme-free group and the non-acidic groups did not show a fluorescence change. However, when the acidic condition was satisfied in the highly pathogenic influenza virus group, both the highly and lowly pathogenic influenza virus groups showed fluorescence changes by treated with both the probe and trypsin, and when furin was treated instead of trypsin, only the highly pathogenic influenza group showed a fluorescence change. Such a result shows that trypsin can be used to detect both the highly and lowly pathogenic influenza viruses and that furin can be used to detect only the lowly pathogenic influenza viruses. Also, even when the enzyme and excreta were treated together, when the acidic condition and enzyme are present together, a fluorescent change was detected, which indicates that the detection capability can be retained even when combined with a different material.

[Experimental Example 3] Confirmation of virus detection effect of probe

A virus detection effect of the probe prepared by the method of Preparation Example 3 was confirmed.

Specifically, the experiment was performed under the same conditions as described in Experimental Example 1, except that the probe of Preparation Example 3 was used, instead of the probe of Preparation Example 3. Results of the measurement of fluorescence intensity are shown in Tables 5 and 6.

[Table 5]

| Sample | pH 5.5 | | pH 7.4 | | Furin + Trypsin | | enzyme-free group | |
|---|---|---|---|---|---|---|---|---|
| | Furin | Trypsin | Furin | Trypsin | pH 5.5 | pH 7.4 | pH 5.5 | pH 7.4 |
| H5N1_01 | 4329 | 3406 | 412 | 594 | 3497 | 536 | 352 | 345 |
| H5N1_02 | 4999 | 4360 | 342 | 381 | 3890 | 552 | 396 | 559 |
| H5N6 | 2251 | 2030 | 328 | 426 | 2198 | 599 | 554 | 596 |
| H1N1_01 | 327 | 3719 | 528 | 361 | 4656 | 321 | 426 | 338 |
| H1N1_02 | 490 | 3152 | 391 | 407 | 4702 | 485 | 381 | 444 |
| H2N1 | 569 | 2064 | 544 | 406 | 2655 | 381 | 503 | 395 |
| H2N4 | 477 | 3458 | 482 | 595 | 3179 | 365 | 380 | 597 |
| H3N8 | 313 | 3397 | 414 | 409 | 4690 | 320 | 588 | 387 |
| H5N2 | 512 | 2671 | 388 | 586 | 2919 | 336 | 556 | 351 |
| H5N3 | 377 | 3571 | 421 | 339 | 3437 | 579 | 478 | 407 |
| H7N9 | 528 | 3249 | 554 | 586 | 3628 | 528 | 311 | 512 |
| H7N7 | 575 | 2513 | 365 | 570 | 2470 | 326 | 577 | 386 |
| H9N2_01 | 461 | 3514 | 375 | 596 | 3182 | 390 | 372 | 391 |
| H9N2_02 | 306 | 3733 | 585 | 420 | 4052 | 317 | 475 | 500 |

[Table 6]

| Sample | enzyme + excreta (pH 5.5) | | Excreta-treated group (enzyme-free) | |
|---|---|---|---|---|
| | Furin | Trypsin | pH 5.5 | pH 7.4 |
| H5N1_01 | 4846 | 4804 | 540 | 308 |
| H5N1_02 | 4335 | 3015 | 447 | 508 |
| H5N6 | 2501 | 2584 | 477 | 317 |
| H1N1_01 | 4964 | 4105 | 520 | 544 |
| H1N1_02 | 3146 | 4577 | 521 | 387 |
| H2N1 | 2286 | 2367 | 533 | 569 |
| H2N4 | 4127 | 3409 | 540 | 454 |
| H3N8 | 4245 | 3828 | 385 | 358 |

(continued)

| Sample | enzyme + excreta (pH 5.5) | | Excreta-treated group (enzyme-free) | |
|---|---|---|---|---|
| | Furin | Trypsin | pH 5.5 | pH 7.4 |
| H5N2 | 2660 | 2614 | 509 | 430 |
| H5N3 | 3157 | 3571 | 464 | 495 |
| H7N9 | 3222 | 4952 | 565 | 577 |
| H7N7 | 2175 | 2493 | 347 | 390 |
| H9N2_01 | 4766 | 4463 | 583 | 450 |
| H9N2_02 | 4607 | 4134 | 381 | 452 |

As shown in Tables 5 and 6, when furin was treated, the enzyme-free group and the non-acidic groups did not show a fluorescence change. However, when the acidic condition was satisfied in the highly pathogenic influenza virus group, and both the highly and lowly pathogenic influenza virus groups showed fluorescence changes by treated with both the probe and trypsin, and when furin was treated instead of trypsin, only the highly pathogenic influenza group showed a fluorescence change. Such a result shows that trypsin can be used to detect both the highly and lowly pathogenic influenza viruses and that furin can be used to detect only lowly pathogenic influenza viruses. Also, even when the enzyme and excreta were treated together, when the acidic condition and enzyme are present together, a fluorescent change was detected, which indicates that the detection capability can be retained even when combined with a different material.

**Claims**

1. A kit for detecting a virus, comprising:

   a biomolecule that specifically reacts with the surface protein of a virus; and
   a probe that reacts with the virus activated by the biomolecule,
   wherein the probe includes a marker bound with an amphiphilic polymer.

2. The kit of claim 1, wherein the marker is one or more selected from the group consisting of a self-quenched dye, a fluorescent dye, an electrochemiluminescent material, a quencher, a luminescent dye and a phosphorescent dye.

3. The kit of claim 1, wherein the probe comprises one or more selected from the group consisting of an amphiphilic polymer-binding dye and an amphiphilic polymer-binding quencher.

4. The kit of claim 1, wherein the amphiphilic polymer is selected from the group consisting of an A-B type block copolymer comprising hydrophilic polymer A and hydrophobic polymer B, a B-A-B type triblock copolymer, a lipid polymer and combinations thereof.

5. The kit of claim 1, wherein the probe is a micelle, a polymersome, a colloidsome, a vesicle, a liposome or a droplet.

6. The kit of claim 1, wherein the virus is influenza virus, coronavirus or paramyxovirus.

7. The kit of claim 1, wherein the biomolecule that specifically reacts with the surface protein of a virus is an enzyme.

8. The kit of claim 7, wherein the enzyme is one or more selected from the group consisting of furin, trypsin, serine, endoprotease and carboxypeptidase.

9. The kit of claim 1, further comprising:

   an acid material with pH 6 or less.

**10.** The kit of claim 1, further comprising:

an adjuvant.

**11.** A composition for detecting a virus, comprising:

a biomolecule that specifically reacts with the surface protein of a virus; and
a probe that reacts with the virus activated by the biomolecule,
wherein the probe includes a marker bound with an amphiphilic polymer.

**12.** A method for detecting a virus, comprising:

contacting a sample obtained from a subject with a biomolecule that specifically reacts with the surface protein of a virus; and
contacting the sample in contact with the biomolecule with a probe that reacts with the virus activated by the biomolecule,
wherein the probe includes a marker bound with an amphiphilic polymer.

**13.** The method of claim 12, wherein the contacts are made under a condition of pH 6 or less.

**14.** The method of claim 12, further comprising:

detecting a change in the probe in contact with the sample.

**15.** The method of claim 14, wherein the change in the probe is detected by measuring one or more selected from the group consisting of fluorescence intensity, luminescence intensity, phosphorescence intensity, absorbance, an electrical signal, a signal from surface-enhanced Raman spectroscopy (SERS), color and dispersity of the probe.

【FIG. 1】

HYDORPHILIC-
HYDROPHOBIC POLYMER

FLUORESCENT DYE

QUENCHER

1.

2.

3.

【FIG. 2】

【FIG. 3】

100 nm

【FIG. 4】

100 nm

【FIG. 5】

## INTERNATIONAL SEARCH REPORT

| International application No. |
|---|
| **PCT/KR2016/000163** |

**A. CLASSIFICATION OF SUBJECT MATTER**

*G01N 33/569(2006.01)i, G01N 33/58(2006.01)i, G01N 21/78(2006.01)i, C12Q 1/37(2006.01)i*

According to International Patent Classification (IPC) or to both national classification and IPC

**B. FIELDS SEARCHED**

Minimum documentation searched (classification system followed by classification symbols)
G01N 33/569; G01N 33/53; G01N 33/58; G01N 21/78; C12Q 1/37

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: virus, detection, diagnosis, influenza, hemagglutinin, enzyme, trypsin

**C. DOCUMENTS CONSIDERED TO BE RELEVANT**

| Category* | Citation of document, with indication, where appropriate, of the relevant passages | Relevant to claim No. |
|---|---|---|
| X | MITTAL, A. et al., "Kinetics of Influenza Hemagglutinin-Mediated Membrane Fusion as a Function of Technique", Analytical Biochemistry, 2002, vol. 303, pages 145-152 See abstract; and pages 145-146. | 1-15 |
| A | GAMBOTTO, A. et al., "Human Infection with Highly Pathogenic H5N1 Influenza Virus", The Lancet, 2008, vol. 371, pages 1464-1475 See the entire document. | 1-15 |
| A | BRANDENBURG, B. et al., "Mechanisms of Hemagglutinin targeted Influenza Virus Neutralization", PloS One, 2013, vol. 8, no. 12, paper number e80034, inner pages 1-14 See the entire document. | 1-15 |
| A | KR 10-2009-0044763 A (REPUBLIC OF KOREA(MANAGEMENT: ANIMAL AND PLANT QUARANTINE AGENCY)) 07 May 2009 See the entire document. | 1-15 |
| A | KR 10-1997-7003432 A (THE MOUNT SINAI SCHOOL OF MEDICINE OF THE CITY UNIVERSITY OF NEW YORK) 03 July 1997 See the entire document. | 1-15 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 APRIL 2016 (14.04.2016) | **14 APRIL 2016 (14.04.2016)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (January 2015)

INTERNATIONAL SEARCH REPORT
Information on patent family members

International application No.

**PCT/KR2016/000163**

| Patent document cited in search report | Publication date | Patent family member | Publication date |
|---|---|---|---|
| KR 10-2009-0044763 A | 07/05/2009 | KR 10-0954992 B1 | 29/04/2010 |
| KR 10-1997-7003432 A | 03/07/1997 | EP 0760013 A1 | 09/08/2000 |
| | | JP 10-500574 A | 20/01/1998 |
| | | JP 3621995 B2 | 23/02/2005 |
| | | US 6150131 A | 21/11/2000 |
| | | WO 95-32309 A1 | 30/11/1995 |

Form PCT/ISA/210 (patent family annex) (January 2015)